# EUROPEAN PATENT SPECIFICATION

(11) **EP 2 840 147 B1**
(45) Date of publication and mention of the grant of the patent: **30.05.2018**
(21) Application number: 13778644.8
(22) Date of filing: 05.04.2013
(51) Int. Cl.: C12Q 1/68, C12N 15/09

(54) **METHOD FOR ASSESSING ENDOMETRIAL CANCER SUSCEPTIBILITY**
VERFAHREN ZUR BEURTEILUNG DER ANFÄLLIGKEIT FÜR ENDOMETRIALES KARZINOM
PROCÉDÉ POUR ESTIMER LA SUSCEPTIBILITÉ AU CANCER DE L'ENDOMÈTRE

(30) Priority: 17.04.2012 JP 2012094198
(43) Date of publication of application: 25.02.2015
(73) Proprietor: Yamaguchi University, Yamaguchi 753-8511 (JP)
(72) Inventor: SUEHIRO, Yutaka, Ube-shi Yamaguchi 755-8505 (JP); SASAKI, Kohsuke, Ube-shi Yamaguchi 755-8505 (JP); OKADA, Takae, Ube-shi Yamaguchi 755-8505 (JP)
(74) Representative: Greenwood, Matthew David
(86) International application number: PCT/JP2013/002361
(87) International publication number: WO 2013/157215

(56) References cited:
- WO-A2-2008/043561
- JP-A- 2008 048 668
- JP-A- 2008 048 668
- CHO ET AL: "Array comparative genomic hybridization analysis of uterine leiomyosarcoma", GYNECOLOGIC ONCOLOGY, ACADEMIC PRESS, LONDON, GB, vol. 99, no. 3, 1 December 2005 (2005-12-01), pages 545-551, XP005166456, ISSN: 0090-8258, DOI: 10.1016/J.YGYNO.2005.07.017
- KEIKO KAWASAKI ET AL.: 'Shikyutaigan ni Okeru Senshokutai 11 Ban Chowan Ryoiki no Kesshitsu to Senshokutai Fuanteisei ni Tsuite no Kento' PROCEEDINGS OF THE JAPANESE CANCER ASSOCIATION vol. 62, 25 August 2003, page 354 (1656-PP), XP008174776
- YUJI IKEDA ET AL.: 'Shikyutaigan ni Okeru K-Ras,NF1 Idenshi Ryoiki no Senshokutai Copy-su Ijo' PROCEEDINGS OF THE JAPANESE CANCER ASSOCIATION vol. 68, 2009, page 134 (P-0155), XP008174777
- A. GUIRGUIS ET AL.: 'Characterization of concurrent ovarian and endometrial carcinoma by copy-number analysis (CNA) and gene expression profiling (GEP), 2007:10546' JOURNAL OF CLINICAL ONCOLOGY, 2007 ASCO ANNUAL MEETING PROCEEDINGS (POST-MEETING EDITION) vol. 25, no. 18S, 2007, XP055165633
- STALO KARAGEORGI ET AL.: 'GSTM1 and GSTT1 Copy Number Variation in Population-based Studies of Endometrial Cancer Risk' CANCER EPIDEMIOLOGY BIOMARKERS AND PREVENTION 2011, XP055164900

## Description

### Technical Field

The present invention relates to a method and a kit for assessing corpus uteri cancer susceptibility.

### Background Art

In Japan, cancer is the leading cause of death. Therefore, cancer control is the highest priority issue from the viewpoint of national health. A basic solution for reducing mortality from cancer is to avoid developing cancer, and "prevention of cancer development and early detection of cancer" are regarded as important. However, because a method for assessing cancer susceptibility (a predisposition to cancer) has not yet been fully established, diagnosis and treatment are more focused on early detection (secondary prevention) than on the prevention of cancer development (primary prevention) under the present circumstances. In light of the above, if susceptibility to various types of cancer can be assessed in each individual in advance, prevention of cancer development, including improving "lifestyles and living environments" etc., can be effectively accomplished, and not only that, current cancer screening will be efficiently and effectively carried out, hopefully increasing the consultation rate.

Corpus uteri cancer is one of the malignant tumors frequently seen in the female genitalia throughout the world. In 2004, 199,000 new-onset cases and 55,000 fatal cases were reported (Non-patent Document 1). Also in Japan, the incidence and death rate of corpus uteri cancer show an increasing trend year by year, with the number of patients in Japan reaching 8,189 in 2009.

Common symptoms of corpus uteri cancer include atypical genital bleeding. As a method for examining corpus uteri cancer in women presented with atypical genital bleeding, endometrial cytodiagnosis and endometrial tissue diagnosis have long been employed as the standard method. Also, corpus uteri cancer is known to be associated with increased endometrial thickness, and transvaginal sonography has been reported as an endometrial hyperplasia examination method (Non-patent Document 2). Further, high expression of factors such as stanniocalcin 2 (STC2) and mutS homolog 2 (MSH2) has been reported in corpus uteri cancer (Patent Document 1), and a method of using these factors as biomarkers of corpus uteri cancer has been proposed.

On the other hand, a DNA copy number polymorphism (CNP) was reported in 2004 as a condition in which DNA duplication or deletion of as many as about 100 kb in size occurs in a specific chromosomal region. In 2008, it was reported that DNA copy number polymorphisms were present in about 20,000 sites in human genome. A DNA copy number polymorphism is a phenomenon in which the number of copies of several thousand base pairs to several million base pairs varies from one individual to another. Normally, human genes are inherited in two copies in total, one copy from the maternal genome and the other copy from the paternal genome. However, it is known that genes are present in only one copy or three or more copies per cell depending on the individual due to DNA copy number polymorphism in the gene region. As a cause of generating constitutional differences among individuals as observed in various degrees of sensitivity to drugs and various levels of manifestation of side effects, "variation in the nucleotide sequence" of human genes has been widely known as represented by Single Nucleotide Polymorphism (SNP). Also, recently, DNA copy number polymorphism has been receiving increasing attention from the aspect of "variation in the number" of genes.

The present inventors have reported an assessment method of corpus uteri cancer susceptibility and colorectal cancer susceptibility using DNA copy number polymorphism as an index (see Patent Document 2). However, according to this report, the chromosomal regions exhibiting the DNA copy number polymorphism specific for corpus uteri cancer patients were merely limited to a nucleotide sequence of approximately 100 kb by screening using BAC arrays, and moreover, later analysis found that the LOC440552 gene region, which was specified by quantitative Polymerase Chain Reaction (PCR) based on the chromosomal regions obtained by the above screening, was derived from mitochondrial DNA. Consequently, a human chromosomal region consisting of a nucleotide sequence of several tens to several hundred bps (base pairs) that exhibits the DNA copy number polymorphism associated with corpus uteri cancer susceptibility has yet to be specified.

### Prior Art Documents

### Patent Documents

Patent Document 1: Japanese unexamined Patent Application Publication No. 2012-21943
Patent Document 2: Japanese unexamined Patent Application Publication No. 2008-48668, (D1), which discloses a method of detecting endometrial cancer rather than susceptibility to that cancer.

### Non-patent Documents

Non-patent Document 1: The global burden of disease: 2004 update, World Health Organization, 2008
Non-patent Document 2: Smith, R. A. et al., CA Cancer J Clin 51: 38 to 75 (2001)
Non-patent Document 3: Cho et al, Gynecologic Oncology Academic Press, Vol 99, no 3, Dec 2005, pp 545-551 (D2) discloses one or more regions in corpus uteri cancer but no specific sequences for detecting susceptibility to that cancer are shown.

### Summary of the Invention

### Object to be Solved by the Invention

An object of the present invention is to provide an assessment method of corpus uteri cancer susceptibility as set out in claim 1. Typically the assessment involves a human chromosomal region consisting of a nucleotide sequence of approximately 100 bps that exhibits a DNA copy number polymorphism associated with corpus
uteri cancer susceptibility is identified and assessment is made based on an increase or decrease in the DNA copy number polymorphism.

### Means to Solve the Object

Using a microarray derived from the peripheral blood, the present inventors obtained 159 chromosomal regions exhibiting the DNA copy number polymorphism specific for corpus uteri cancer by screening. Subsequently, the present inventors searched for a human chromosomal region consisting of a nucleotide sequence of approximately 100 bps that exhibits the DNA copy number polymorphism specific for corpus uteri cancer from a vast number of chromosomal regions including not only the aforementioned 159 regions, but also regions not covered by the microarray, based on their long years of experience and instincts. As a result, they have found that DNA copy number in a human chromosomal region consisting of a nucleotide sequence shown in any of SEQ ID NOs: 1 to 7 was significantly decreased in corpus uteri cancer patients in comparison with healthy individuals, thereby completing the present invention.

That is, the present invention relates to (1) an assessment of corpus uteri cancer susceptibility, comprising detecting a decrease in DNA copy number in a human chromosomal region consisting of a nucleotide sequence shown in any of SEQ ID NOs: 1 to 7, (2) the assessment method according to the aforementioned (1), comprising performing discriminant analysis by selecting two or more of DNA in a human chromosomal region consisting of a nucleotide sequence shown in any of SEQ ID NOs: 1 to 7, (3) the assessment method according to the aforementioned (2), comprising selecting three DNAs consisting of nucleotide sequences shown respectively in SEQ ID NO: 2, SEQ ID NO: 3, and SEQ ID NO: 6, and (4) the assessment method according to any one of the aforementioned (1) to (3), wherein the decrease in DNA copy number is detected by quantitative PCR.

The aforementioned assessment methods of the present invention do not include a diagnostic act performed by a doctor, and other aspects of the present invention include a method for collecting data for the assessment of corpus uteri cancer susceptibility and a method for collecting data for the prediction of the prognosis of corpus uteri cancer treatment.

The present invention also relates to (5) a kit for assessing corpus uteri cancer susceptibility, comprising a primer set or probe, or a labeled primer set or probe for detecting a decrease in DNA copy number in a human chromosomal region consisting of a nucleotide sequence shown in any of SEQ ID NOs: 1 to 7.

### Effect of the Invention

The present invention enables assessment of a predisposition to corpus uteri cancer (corpus uteri cancer susceptibility [risk of developing corpus uteri cancer]) by detecting DNA copy number in a chromosomal region exhibiting the DNA copy number polymorphism specific for corpus uteri cancer. Because the chromosomal region exhibiting the DNA copy number polymorphism specific for corpus uteri cancer according to Patent Document 2 has a base length of as long as approximately 100 kbps, this region can only be detected by microarray assay, which has drawbacks in terms of cost-effectiveness and time-effectiveness. In contrast, because the chromosomal region exhibiting the DNA copy number polymorphism specific for corpus uteri cancer according to the present invention has a base length of as short as 75 bps to 111 bps, this region can be detected by quantitative PCR, which is excellent in terms of not only cost-effectiveness and time-effectiveness, but also sensitivity and simplicity.

### Brief Description of Drawings

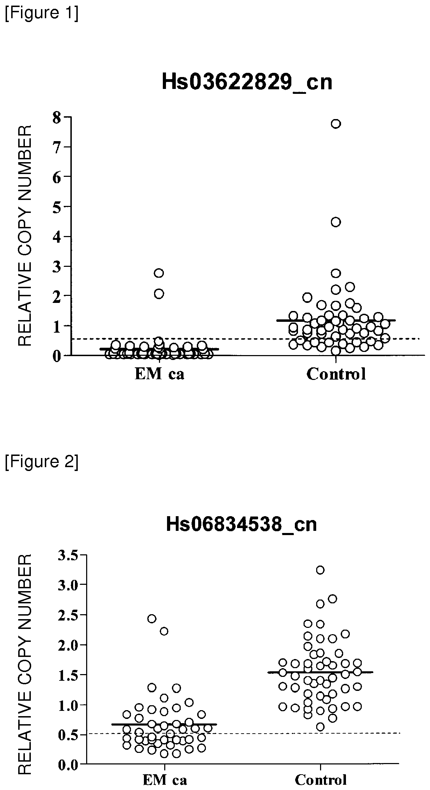

[Figure 1] Figure 1 is a diagram showing the results of detection of the DNA copy number by quantitative PCR with the primer and probe set (Hs03622829_cn) in the group of healthy individuals (Control) and the group of corpus uteri cancer patients (EM ca). The horizontal line indicates the mean value in each group. The dotted line indicates the cut-off value. The symbol ○ indicates the number of copies in each individual.
[Figure 2] Figure 2 is a diagram showing the results of detection of the DNA copy number by quantitative PCR with the primer and probe set (Hs06834538_cn) in the group of healthy individuals (Control) and the group of corpus uteri cancer patients (EM ca). The horizontal line indicates the mean value in each group. The dotted line indicates the cut-off value. The symbol ○ indicates the number of copies in each individual.

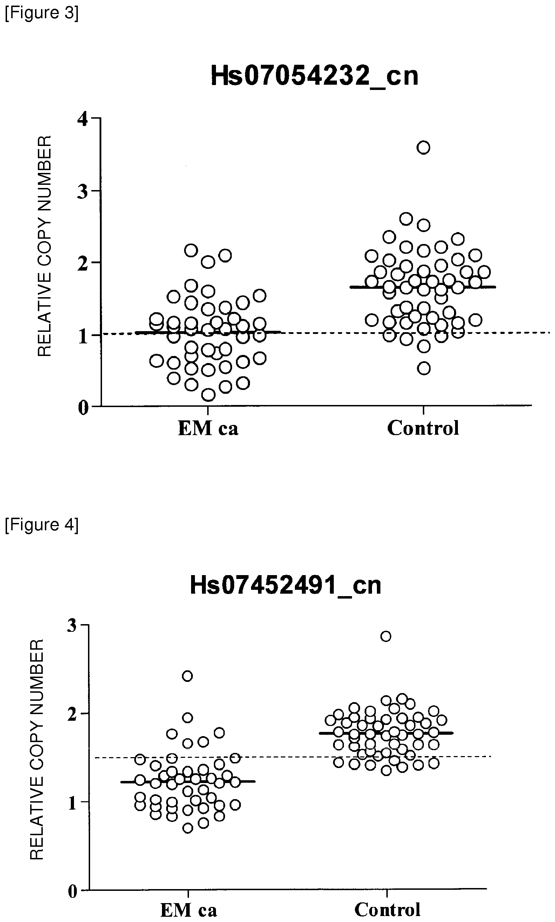

[Figure 3] Figure 3 is a diagram showing the results of detection of the DNA copy number by quantitative PCR with the primer and probe set (Hs07054232_cn) in the group of healthy individuals (Control) and the group of corpus uteri cancer patients (EM ca). The horizontal line indicates the mean value in each group. The dotted line indicates the cut-off value. The symbol ○ indicates the number of copies in each individual.
[Figure 4] Figure 4 is a diagram showing the results of detection of the DNA copy number by quantitative PCR with the primer and probe set (Hs07452491_cn) in the group of healthy individuals (Control) and the group of corpus uteri cancer patients (EM ca). The horizontal line indicates the mean value in each group. The dotted line indicates the cut-off value. The symbol ○ indicates the number of copies in each individual.

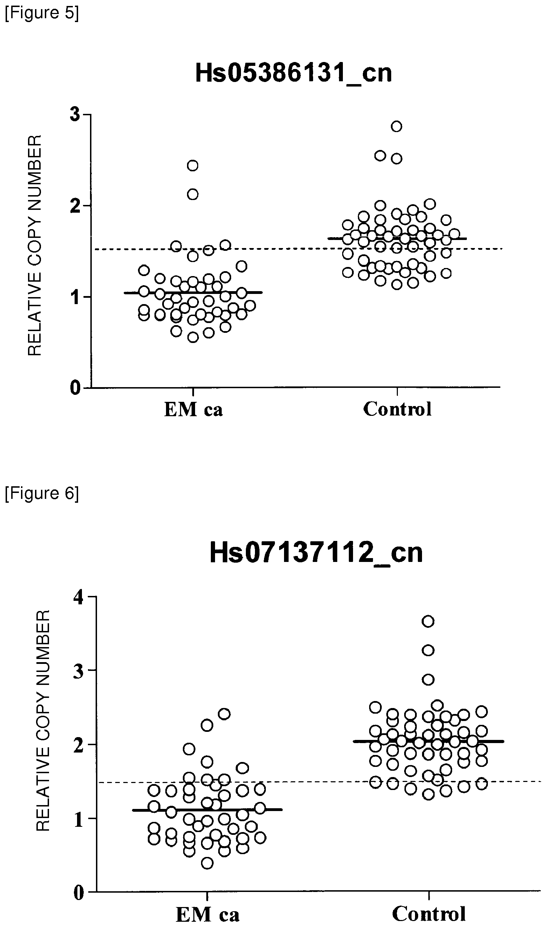

[Figure 5] Figure 5 is a diagram showing the results of detection of the DNA copy number by quantitative PCR with the primer and probe set (Hs05386131_cn) in the group of healthy individuals (Control) and the group of corpus uteri cancer patients (EM ca). The horizontal line indicates the mean value in each group. The dotted line indicates the cut-off value. The symbol ○ indicates the number of copies in each individual.
[Figure 6] Figure 6 is a diagram showing the results of detection of the DNA copy number by quantitative PCR with the primer and probe set (Hs07137112_cn) in the group of healthy individuals (Control) and the group of corpus uteri cancer patients (EM ca). The horizontal line indicates the mean value in each group. The dotted line indicates the cut-off value. The symbol ○ indicates the number of copies in each individual.

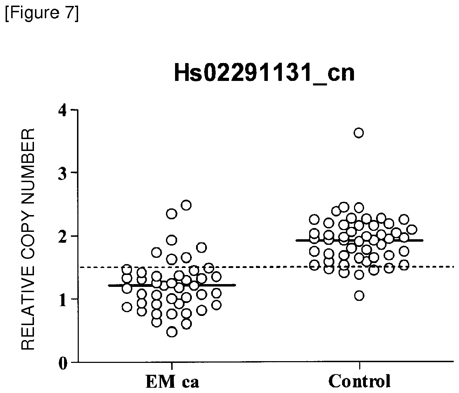

[Figure 7] Figure 7 is a diagram showing the results of detection of the DNA copy number by quantitative PCR with the primer and probe set (Hs02291131_cn) in the group of healthy individuals (Control) and the group of corpus uteri cancer patients (EM ca). The horizontal line indicates the mean value in each group. The dotted line indicates the cut-off value. The symbol ○ indicates the number of copies in each individual.

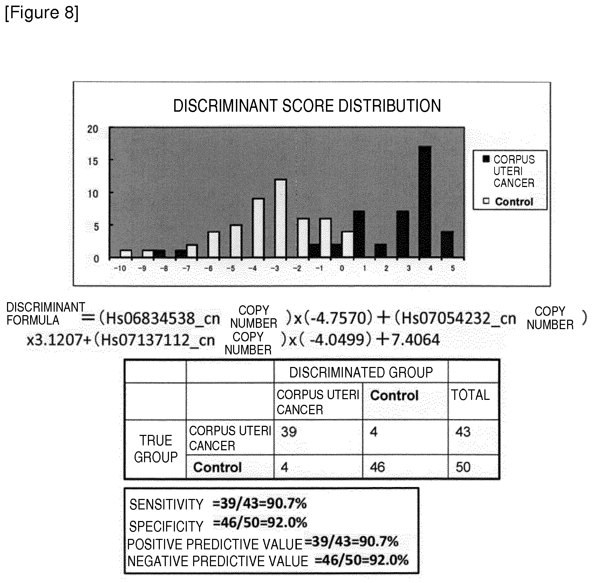

[Figure 8] Figure 8 is a diagram showing the results of the discriminant analysis performed based on the results obtained in Figures 2, 3 and 6.

### Mode of Carrying Out the Invention

The assessment method of corpus uteri cancer susceptibility of the present invention is not particularly limited as long as it is a method for detecting a decrease in DNA copy number in human chromosomal region consisting of a nucleotide sequence shown in any of SEQ ID NOs: 1 to 7 having a base length of 75 bps- 111 bps (with the proviso that the diagnostic act performed by a doctor is excluded) and the kit for assessing corpus uteri cancer susceptibility of the present invention is not particularly limited as long as it is a kit comprising a primer set or probe, or a labeled primer set or probe for the detection of a decrease in DNA copy number in a human chromosomal region consisting of a nucleotide sequence shown in any of SEQ ID NOs: 1 to 7 having a base length of 75 bps - 111 bps. Here, the term "decrease in copy number" refers to a condition in which DNA copy number in a specific chromosomal region in an individual subjected to assessment is decreased compared to a healthy individual. Also, the term "detection of a decrease in DNA copy number" refers to detecting the degree of decrease in DNA copy number and the frequency of decrease in DNA copy number. Here, a "decrease in DNA copy number" also encompasses a "decrease in gene copy number."

DNA in a human chromosomal region consisting of a nucleotide sequence shown in any of SEQ ID NOs: 1 to 7 of the present invention can also be specified as a region based on the human chromosomal location information provided in "NCBI; Feb 2009 human reference sequence (GRCh37/hg19)." Specifically, DNA in a human chromosomal region consisting of a nucleotide sequence shown in SEQ ID NO: 1 can be specified as 7p22.3 (1,275,532 to 1,275,641), DNA in a human chromosomal region consisting of a nucleotide sequence shown in SEQ ID NO: 2 as 9q22.32 (98,277,504 to 98,277,595), DNA in a human chromosomal region consisting of a nucleotide sequence shown in SEQ ID NO: 3 as 14q22.1 (51,296,210 to 51,296,320), DNA in a human chromosomal region consisting of a nucleotide sequence shown in SEQ ID NO: 4 as 15q22.2 (59,732,253 to 59,732,342), DNA in a human chromosomal region consisting of a nucleotide sequence shown in SEQ ID NO: 5 as 15q26.1 (93,631,100 to 93,631,174), DNA in a human chromosomal region consisting of a nucleotide sequence shown in SEQ ID NO: 6 as 19q12 (30,437,054 to 30,437,155), and DNA in a human chromosomal region consisting of a nucleotide sequence shown in SEQ ID NO: 7 as 21q22.13 (38,122,020 to 38,122,129). When there is a change in the human chromosomal location information by a version update and the like, the corresponding chromosomal region can be appropriately selected.

Examples of the aforementioned method for detecting a decrease in DNA copy number include a detection method using microarray assay, quantitative polymerase chain reaction (PCR), loop-mediated isothermal amplification (LAMP), fluorescence in situ hybridization (FISH), and smart amplification process (SMAP), using human blood or human tissues as a detection sample. When detection is performed with high throughput, microarray assay is preferable, while when a decrease in DNA copy number is detected with high precision, quantitative PCR is preferable.

The microarray (microchip) used in the aforementioned microarray assay is a microarray (microchip) in which probes consisting of human chromosome fragments are fixed in a predetermined area on a support. The support of a microarray (microchip) may be any one that can be used for hybridization, and for example, a substrate such as glass, silicone, and plastic, a nitrocellulose membrane, and a nylon membrane are preferably used. While the microarray (microchip) can be produced by any method publicly known to those skilled in the art, a commercially available product can also be used. Examples of the commercially available product include 2.1M Array (R) manufactured by Roche, MAC Array (R) manufactured by Macrogen, Inc., and SpectralChip (R) manufactured by SPECTRAL GENOMICS, Inc. Among them, 2.1M Array (R) manufactured by Roche can be given as a preferable example.

The aforementioned hybridization may be carried out, for example, to allow chromosomal DNA that is labeled with a fluorescent substance such as Cy-3 and Cy-5 in advance to hybridize to probes fixed to the surface of the microarray (microchip). The hybridization conditions may be appropriately selected from Molecular cloning, A laboratory manual, second edition, pp. 9.52 to 9.55 (1989), and the like.

Examples of the aforementioned quantitative PCR include competitive PCR and real-time PCR, of which real-time PCR is preferable due to its high versatility. Examples of a method for detecting DNA amplified in real-time PCR include the TaqMan method using a fluorescent dye-conjugated probe (TaqMan probe) (Japanese Patent No. 2825976) and the intercalater method using an intercalater such as SYBR (R) Green I. Among them, the TaqMan method is preferable since it achieves higher specificity due to the specificity of primers combined with the specificity of probes. Real-time PCR can be performed using a specialized device for real-time PCR in which a common thermal cycler and a common spectrophotofluorometer are integrated.

In the assessment method of corpus uteri cancer susceptibility according to the present invention, as long as the primer set for the detection of a decrease in the DNA copy number by quantitative PCR is capable of amplifying the chromosomal region to be detected, the length of the primer sequence, the annealing site in the human chromosomal region, and the like can be appropriately selected in consideration of DNA amplification efficiency and specificity. In order to detect a decrease in DNA copy number in a human chromosomal region consisting of a nucleotide sequence shown in any of SEQ ID NOs: 1 to 7 of the present invention, for example, a primer and probe set manufactured by Applied Biosystems, which includes a set of a DNA amplification primer set and a detection probe for TaqMan assay, can be obtained by searching its product number in, for example, the website of Applied Biosystems (http://www5.appliedbiosystems.com/tools/cnv/) and ordering it. As specific examples, DNA in a human chromosomal region consisting of a nucleotide sequence shown in SEQ ID NO: 1 can be detected by the primer and probe set "Hs03622829_cn", DNA in a human chromosomal region consisting of a nucleotide sequence shown in SEQ ID NO: 2 by the primer and probe set "Hs06834538_cn", DNA in a human chromosomal region consisting of a nucleotide sequence shown in SEQ ID NO: 3 by the primer and probe set "Hs07054232_cn", DNA in a human chromosomal region consisting of a nucleotide sequence shown in SEQ ID NO: 4 by the primer and probe set "Hs07452491_cn", DNA in a human chromosomal region consisting of a nucleotide sequence shown in SEQ ID NO: 5 by the primer and probe set "Hs05386131_cn", DNA in a human chromosomal region consisting of a nucleotide sequence shown in SEQ ID NO: 6 by the primer and probe set "Hs07137112_cn", and DNA in a human chromosomal region consisting of a nucleotide sequence shown in SEQ ID NO: 7 by the primer and probe set "Hs02291131_cn." Also, the internal control primer and probe set "RNaseP", which is for detecting DNA in a human chromosomal region consisting of a nucleotide sequence shown in SEQ ID NO: 8, can be used concurrently.

Examples of the aforementioned LAMP can include a method involving mixing materials such as a plurality of primers for amplifying a specific gene region, template DNA, strand-displacing DNA polymerase, and dNTP, allowing reactions to proceed at a certain temperature (around 65°C) for a certain period of time, and then detecting the amplification based on the turbidity of the reaction solution.

Examples of the aforementioned FISH can include a technique involving performing hybridization between probe DNA, which has a DNA sequence capable of hybridizing to the target genomic region in a chromosome preparation and is labeled with a fluorescent substance such as fluorescein isothiocyanate (FITC), tetramethyl rhodamin isothiocyanate (TRITC), CyDye (R), and a chromosomal region, and counting the quantity of fluorescent signal resulting from the hybridization under a fluorescent microscope. Also, for DNA present on the same chromosome as the target region, an aberration in copy number can be more accurately evaluated by simultaneously performing hybridization using probes labeled with different kinds of fluorescence.

Examples of the aforementioned SMAP include a method involving mixing materials such as a plurality of primers for amplifying a specific gene region, template DNA, strand-displacing DNA polymerase, and dNTP, allowing reactions to proceed at a certain temperature (around 60°C) for a certain period of time, and then detecting the amplification based on the fluorescent intensity of the reaction solution.

In order to increase the precision of the assessment method of the present invention, it is preferable to perform discriminant analysis by selecting two or more regions from DNA in a human chromosomal region consisting of a nucleotide sequence shown in any of SEQ ID NOs: 1 to 7. The number and type of regions to be selected can be appropriately selected in consideration of sensitivity (the probability of patients in a group of true corpus uteri cancer patients being tested positive) [assessed as susceptible to corpus uteri cancer]) and specificity (the probability of healthy individuals in a group of true healthy individuals being assessed as tested negative [assessed as not susceptible to corpus uteri cancer]), and/or positive predictive value (the probability of individuals in a group of individuals tested positive [assessed as susceptible to corpus uteri cancer] truly having corpus uteri cancer) and negative predictive value (the probability of individuals in a group of individuals tested negative [assessed as not susceptible to corpus uteri cancer] being true healthy individuals). Sensitivity and specificity may both be at least 50%, can both be preferably at least 80%, more preferably at least 85%, and most preferably at least 90%, and positive predictive value and negative predictive value may both be at least 50%, can both be preferably at least 80%, more preferably at least 85%, and most preferably at least 90%. Sensitivity (%) can be calculated by inputting into the formula "([number of individuals tested positive in a group of true corpus uteri cancer patients] / [number of true corpus uteri cancer patients])× 100", specificity (%) by the formula "([number of individuals tested negative in a group of true healthy individuals] / [number of true healthy individuals]) × 100, positive predictive value (%) by the formula "([number of true corpus uteri cancer patients in a group of individuals tested positive] / [number of individuals tested positive]) × 100", and negative predictive value (%) by the formula "([number of true healthy individuals in a group of individuals tested negative] / [number of individuals tested negative]) × 100."

Also, in the assessment method of the present invention, DNA to be selected preferably includes at least DNA in a human chromosomal region consisting of a nucleotide sequence shown in SEQ ID NO: 2. Specific combinations of DNAs in the case when regions selected contain DNA in a human chromosomal region consisting of a nucleotide sequence shown in SEQ ID NO: 2 can include a combination of two DNAs consisting of nucleotide sequences shown respectively in SEQ ID NO: 2 and SEQ ID NO: 1, a combination of two DNAs consisting of nucleotide sequences shown respectively in SEQ ID NO: 2 and SEQ ID NO: 3, a combination of two DNAs consisting of nucleotide sequences shown respectively in SEQ ID NO: 2 and SEQ ID NO: 4, a combination of two DNAs consisting of nucleotide sequences shown respectively in SEQ ID NO: 2 and SEQ ID NO: 5, a combination of two DNAs consisting of nucleotide sequences shown respectively in SEQ ID NO: 2 and SEQ ID NO: 6, a combination of two DNAs consisting of nucleotide sequences shown respectively in SEQ ID NO: 2 and SEQ ID NO: 7, a combination of three DNAs consisting of nucleotide sequences shown respectively in SEQ ID NO: 2, SEQ ID NO: 1, and SEQ ID NO: 3, a combination of three DNAs consisting of nucleotide sequences shown respectively in SEQ ID NO: 2, SEQ ID NO: 1, and SEQ ID NO: 4, a combination of three DNAs consisting of nucleotide sequences shown respectively in SEQ ID NO: 2, SEQ ID NO: 1, and SEQ ID NO: 5, a combination of three DNAs consisting of nucleotide sequences shown respectively in SEQ ID NO: 2, SEQ ID NO: 1, and SEQ ID NO: 6, a combination of three DNAs consisting of nucleotide sequences shown respectively in SEQ ID NO: 2, SEQ ID NO: 1, and SEQ ID NO: 7, a combination of three DNAs consisting of nucleotide sequences shown respectively in SEQ ID NO: 2, SEQ ID NO: 3, and SEQ ID NO: 4, a combination of three DNAs consisting of nucleotide sequences shown respectively in SEQ ID NO: 2, SEQ ID NO: 3, and SEQ ID NO: 5, a combination of three DNAs consisting of nucleotide sequences shown respectively in SEQ ID NO: 2, SEQ ID NO: 3, and SEQ ID NO: 6, a combination of three DNAs consisting of nucleotide sequences shown respectively in SEQ ID NO: 2, SEQ ID NO: 3, and SEQ ID NO: 7, a combination of three DNAs consisting of nucleotide sequences shown respectively in SEQ ID NO: 2, SEQ ID NO: 4, and SEQ ID NO: 5, a combination of three DNAs consisting of nucleotide sequences shown respectively in SEQ ID NO: 2, SEQ ID NO: 4, and SEQ ID NO: 6, a combination of three DNAs consisting of nucleotide sequences shown respectively in SEQ ID NO: 2, SEQ ID NO: 4, and SEQ ID NO: 7, a combination of three DNAs consisting of nucleotide sequences shown respectively in SEQ ID NO: 2, SEQ ID NO: 5, and SEQ ID NO: 6, a combination of three DNAs consisting of nucleotide sequences shown respectively in SEQ ID NO: 2, SEQ ID NO: 5, and SEQ ID NO: 7, a combination of three DNAs consisting of nucleotide sequences shown respectively in SEQ ID NO: 2, SEQ ID NO: 6, and SEQ ID NO: 7, a combination of four DNAs consisting of nucleotide sequences shown respectively in SEQ ID NO: 2, SEQ ID NO: 1, SEQ ID NO: 3, and SEQ ID NO: 4, a combination of four DNAs consisting of nucleotide sequences shown respectively in SEQ ID NO: 2, SEQ ID NO: 1, SEQ ID NO: 3, and SEQ ID NO: 5, a combination of four DNAs consisting of nucleotide sequences shown respectively in SEQ ID NO: 2, SEQ ID NO: 1, SEQ ID NO: 3, and SEQ ID NO: 6, a combination of four DNAs consisting of nucleotide sequences shown respectively in SEQ ID NO: 2, SEQ ID NO: 1, SEQ ID NO: 3, and SEQ ID NO: 7, a combination of four DNAs consisting of nucleotide sequences shown respectively in SEQ ID NO: 2, SEQ ID NO: 3, SEQ ID NO: 4, and SEQ ID NO: 5, a combination of four DNAs consisting of nucleotide sequences shown respectively in SEQ ID NO: 2, SEQ ID NO: 3, SEQ ID NO: 4, and SEQ ID NO: 6, a combination of four DNAs consisting of nucleotide sequences shown respectively in SEQ ID NO: 2, SEQ ID NO: 3, SEQ ID NO: 4, and SEQ ID NO: 7, a combination of four DNAs consisting of nucleotide sequences shown respectively in SEQ ID NO: 2, SEQ ID NO: 3, SEQ ID NO: 5, and SEQ ID NO: 6, a combination of four DNAs consisting of nucleotide sequences shown respectively in SEQ ID NO: 2, SEQ ID NO: 3, SEQ ID NO: 5, and SEQ ID NO: 7, a combination of four DNAs consisting of nucleotide sequences shown respectively in SEQ ID NO: 2, SEQ ID NO: 3, SEQ ID NO: 6, and SEQ ID NO: 7, a combination of five DNAs consisting of nucleotide sequences shown respectively in SEQ ID NO: 2, SEQ ID NO: 1, SEQ ID NO: 3, SEQ ID NO: 4, and SEQ ID NO: 5, a combination of five DNAs consisting of nucleotide sequences shown respectively in SEQ ID NO: 2, SEQ ID NO: 1, SEQ ID NO: 3, SEQ ID NO: 4, and SEQ ID NO: 6, a combination of five DNAs consisting of nucleotide sequences shown respectively in SEQ ID NO: 2, SEQ ID NO: 1, SEQ ID NO: 3, SEQ ID NO: 4, and SEQ ID NO: 7, a combination of five DNAs consisting of nucleotide sequences shown respectively in SEQ ID NO: 2, SEQ ID NO: 1, SEQ ID NO: 3, SEQ ID NO: 5, and SEQ ID NO: 6, a combination of five DNAs consisting of nucleotide sequences shown respectively in SEQ ID NO: 2, SEQ ID NO: 1, SEQ ID NO: 3, SEQ ID NO: 5, and SEQ ID NO: 7, a combination of five DNAs consisting of nucleotide sequences shown respectively in SEQ ID NO: 2, SEQ ID NO: 1, SEQ ID NO: 3, SEQ ID NO: 6, and SEQ ID NO: 7, a combination of six DNAs consisting of nucleotide sequences shown respectively in SEQ ID NO: 2, SEQ ID NO: 1, SEQ ID NO: 3, SEQ ID NO: 4, SEQ ID NO: 5, and SEQ ID NO: 6, a combination of six DNAs consisting of nucleotide sequences shown respectively in SEQ ID NO: 2, SEQ ID NO: 1, SEQ ID NO: 3, SEQ ID NO: 4, SEQ ID NO: 5, and SEQ ID NO: 7, a combination of six DNAs consisting of nucleotide sequences shown respectively in SEQ ID NO: 2, SEQ ID NO: 3, SEQ ID NO: 4, SEQ ID NO: 5, SEQ ID NO: 6, and SEQ ID NO: 7, and a combination of seven DNAs consisting of nucleotide sequences shown respectively in SEQ ID NO: 2, SEQ ID NO: 1, SEQ ID NO: 3, SEQ ID NO: 4, SEQ ID NO: 5, SEQ ID NO: 6, and SEQ ID NO: 7. Among them, a combination of three DNAs consisting of nucleotide sequences shown respectively in SEQ ID NO: 2, SEQ ID NO: 3, and SEQ ID NO: 6 is preferable.

The aforementioned discriminant analysis is not limited as long as it is a technique for obtaining a function (discriminant function) to determine as to, based on the data showing which sample (detection sample) belongs to which group (a group of healthy individuals or a group of corpus uteri cancer patients) prepared in advance, to which group a sample belongs, when the group to which the sample belongs is unknown. Among the discriminant functions, examples of a linear discriminant function include a hyperplane/linear discriminant function and examples of a non-linear discriminant function include a hypersurface/curved discriminant function based on the generalized Mahalanobis distance.

In the following case, an individual subjected to assessment can be assessed as highly susceptible to corpus uteri cancer (having a high risk of developing corpus uteri cancer): A significant decrease in DNA copy number is noted in an individual subjected to assessment as a result of detecting DNA copy number in a human chromosomal region consisting of a nucleotide sequence shown in any of SEQ ID NOs: 1 to 7 of the present invention in each of the individual subjected to assessment and a healthy individual (control) and comparing the resulting DNA copy numbers between these individuals. Also, the method for collecting data for the assessment of corpus uteri cancer susceptibility and the method for collecting data for the prediction of the prognosis of corpus uteri cancer treatment of the present invention include the step of collecting the results of detection of DNA copy numbers in an individual subjected to assessment and a healthy individual (control) as data.

With regard to a primer set in the kit for assessing corpus uteri cancer susceptibility of the present invention, as long as it is a complementary primer set capable of annealing to a part of the upstream and downstream sequences of DNA in a human chromosomal region consisting of a nucleotide sequence shown in any of SEQ ID NOs: 1 to 7 of the present invention, the length of the primer sequence, the annealing site in the human chromosomal region, the length of DNA to be amplified, and the like can be appropriately selected in consideration of DNA amplification efficiency and specificity. Also, the aforementioned assessment kit can include the internal control primer and probe set "RNaseP", which is for detecting DNA in a human chromosomal region consisting of a nucleotide sequence shown in SEQ ID NO: 8.

The probe in the kit for assessing corpus uteri cancer susceptibility of the present invention may be a probe hybridizing to all or a part of DNA in a human chromosomal region consisting of a nucleotide sequence shown in any of SEQ ID NOs: 1 to 7 of the present invention. Examples of the label attached to the aforementioned probe can include biotin, fluorescein, and ³²P.

Hereinbelow, the present invention will be described more specifically with reference to Examples. However, the technical scope of the present invention is not limited to these exemplifications.

### Examples

### Example 1

### 1. Screening for a DNA copy number polymorphic region associated with corpus uteri cancer susceptibility by microarray assay

### 1-1 Materials

[1] DNA (20 samples) extracted from the peripheral blood of 20 healthy females (Group of healthy individuals)
[2] DNA (20 samples) extracted from the peripheral blood of 20 sporadic corpus uteri cancer patients (Group of corpus uteri cancer patients)
[3] DNA pool, which contains DNA extracted from the peripheral blood of 20 healthy females combined in one tube (Reference DNA)

### 1-2 Method

### 1-2-1 Fluorescent labeling of DNA (Nimblegen Dual-Color DNA Labeling Kit [Roche] was used according to the product protocol)

Cy3-Random Nonamer and Cy5-Random Nonamer are each diluted by adding 998.25 µl of a Random Primer Buffer and 1.75 µl of β mercaptoethanol in advance.

### (1) Labeling of test DNA

[1] Prepare two 0.5 ml tubes and place the following materials in each tube:
   Test DNA (1 µg) (DNA derived from the peripheral blood of corpus uteri cancer patients or healthy females),
   Diluted Cy-3-Random Nonamers (40 µl), and
   Nuclease-free water (in an amount to bring the total volume to 80 µl).
[2] Incubate at 98°C for 10 minutes, and then for 2 minutes on ice.
[3] Add the following reagents to each tube:
   10 mM dNTP Mix (10 µl),
   Nuclease-free water (8 µl), and
   50 U/µl Klenow Fragment (2 µl).
[4] Incubate at 37°C overnight.
[5] Add 10 µl of a stop solution (0.5 M EDTA) to each tube.
[6] Add 11.5 µl of 5M NaCl to each tube.
[7] Add 110 µl of isopropanol to each tube.
[8] Combine the contents of the two tubes in one 1.5 ml tube.
[9] After thoroughly mixing, incubate at room temperature for 10 minutes.
[10] After centrifuging at 12,000 g for 10 minutes, pipet out the supernatant.
[11] After adding 500 µl of cooled 80% ethanol to each tube, centrifuge at 12,000 g for 2 minutes, and pipet out the supernatant.
[12] Naturally dry DNA in a light-shielded environment to pelletize it.
[13] Store the resulting DNA at -20°C.

### (2) Labeling of reference DNA

[1] Prepare two 0.5 ml tubes and place the following materials in each tube:
   Reference DNA (1 µg) (DNA pool derived from the peripheral blood of 30 healthy females),
   Diluted Cy-5-Random Nonamers (40 µl), and
   Nuclease-free water (in an amount to bring the total volume to 80 µl).
[2] Incubate at 98°C for 10 minutes, and then for 2 minutes on ice.
[3] Add the following reagents to each tube:
   10 mM dNTP Mix (10 µl),
   Nuclease-free water (8 µl), and
   50 U/µl Klenow Fragment (2 µl).
[4] Incubate at 37°C overnight.
[5] Add 10 µl of a stop solution (0.5 M EDTA) to each tube.
[6] Add 11.5 µl of 5M NaCl to each tube.
[7] Add 110 µl of isopropanol to each tube.
[8] Combine the contents of the two tubes in one 1.5 ml tube.
[9] After thoroughly mixing, incubate at room temperature for 10 minutes.
[10] After centrifuging at 12,000 g for 10 minutes, pipet out the supernatant.
[11] After adding 500 µl of cooled 80% ethanol to each tube, centrifuge at 12,000 g for 2 minutes, and pipet out the supernatant.
[12] Naturally dry DNA in a light-shielded environment to pelletize it.
[13] Store the resulting DNA at -20°C.

### 1-2-2 Hybridization

[1] After adding 20 µl of purified water to each pellet, leave the pellet for 20 minutes, followed by vortexing.
[2] Measure the DNA concentration.
[3] Place the following materials into a 0.5 ml tube:
   Fluorescently-labeled test DNA (34 µg),
   Fluorescently-labeled reference DNA (34 µg), and
   purified water (in an amount to bring the total volume to 12.3 µl).
[4] Place the following reagents included in the NimbleGen Hybridization Kit (manufactured by Roche) in a 0.5 ml tube:
   2× Hybridization Buffer (29.5 µl),
   Hybridization Component A (11.8 µl), and
   Alignment Oligo (1.2 µl).
[5] Add 31.7 µl of the solution prepared in [4] into the solution prepared in [3].
[6] Incubate at 95°C for 5 minutes, then at 42°C for 5 minutes or longer.
[7] Set the 2.1M array (manufactured by Roche) in the Nimblegen HX1 Mixer (manufactured by Roche) and place the array on a heat block at 42°C.
[8] Pour 41 µl of the solution prepared in [5] into the 2.1M array.
[9] Set the 2.1M array slide in the Nimblegen hybridization system (manufactured by Roche) and carry out hybridization for 72 hours.

### 1-2-3 Washing (using the NimbleGen Wash Buffer Kit and the NimbleGen Array Processing Accessories (manufactured by Roche))

[1] Prepare Wash solutions 1, 2, and 3.
   1) Wash solution 1 (2 sets)
      Water (manufactured by VWR) (225 ml)
      10× Wash Buffer I (25 ml)
      1M DTT (25 µl)
   2) Wash solution 2 (1 set)
      Water (manufactured by VWR) (225 ml)
      10× Wash Buffer II (25 ml)
      1M DTT (25 µl)
   3) Wash solution 3 (1 set)
      Water (manufactured by VWR) (225 ml)
      10× Wash Buffer III (25 ml)
      1M DTT (25 µl)
[2] Immerse the 2.1M array in Wash solution 1 that has been warmed to 40°C in advance to wash out the Nimblegen HX1 Mixer.
[3] Shake the 2.1M array in Wash solution 1 for 10 to 15 seconds to wash out the hybridization buffer.
[4] Wash the 2.1M array in Wash solution 1 (room temperature) by thoroughly shaking for 2 minutes.
[5] Wash the 2.1M array in Wash solution 2 (room temperature) by thoroughly shaking for one minute.
[6] Wash the 2.1M array in Wash solution 3 (room temperature) by thoroughly shaking for 15 seconds.
[7] Dry the slide using a centrifuge.

### 1-2-4 Scanning of the array slide and data analysis

[1] Set the 2.1M array in the microarray scanner GenePix 4000B (manufactured by Axon instruments) and scan the fluorescence of the array.
[2] Analyze the fluorescence image file thus obtained by the NimbleScan v2.5 software (manufactured by Roche) to obtain the fluorescence intensity information of each probe in the 2.1M array.
[3] Compare the copy number polymorphic regions between healthy individuals and corpus uteri cancer patients by the Nexus copy number software (version 5, manufactured by BioDiscovery, Inc.) to screen the CNP region associated with the development of corpus uteri cancer.

### 1-3 Results

In comparing the group of corpus uteri cancer patients with the group of healthy individuals, CNP regions in which [1] the copy number alteration occurred at a frequency of 25% or more in either group, and [2] there was a statistically significant difference in the frequency of copy number alteration (P < 0.05 by the Fisher's test) between the above two groups were identified at 159 sites.

### Example 2

### 2. Identification of CNP enabling the prediction of corpus uteri cancer susceptibility by quantitative PCR

In order to identify CNP enabling the prediction of corpus uteri cancer susceptibility, analysis was undertaken using the 159 CNP regions screened by the microarray assay and regions close to them by quantitative PCR. Also, as a control, the number of copies of the ribonuclease P (RNase P) (SEQ ID NO: 8) gene region on chromosome 14 was quantitated.

### 2-1 Materials

[1]: DNA (50 samples) extracted from the peripheral blood of healthy females (Group of healthy individuals)
[2]: DNA (43 samples) extracted from the peripheral blood of corpus uteri cancer patients (females) (Group of corpus uteri cancer patients)
[3]: DNA pool containing DNA extracted from the peripheral blood of 20 healthy females prepared in 1-1 [3] (Reference DNA)

### 2-2 Method

### [1] Add the following reagents:

5 ng/µl DNA (2 µl),
TaqMan Genotyping Master Mix (manufactured by Applied Biosystems) (5 µl),
TaqMan Copy Number Assay primer and probe set (manufactured by Applied Biosystems) (0.5 µl),
TaqMan Copy Number Reference Assay RNase P (manufactured by Applied Biosystems) (0.5 µl), and
Purified water (2 µl)

With respect to the PCR primers and probes, predesigned products were searched in the website of Applied Biosystems (http://www5.appliedbiosystems.com/tools/cnv/) and used.

### [2] Quantitative PCR

Using the 7900HT Fast Real-Time PCR System (manufactured by Applied Biosystems), PCR reactions were carried out as shown in the following Table 1.

**[Table 1]**

| Stage | Temperature | Time |
|---|---|---|
| Hold | 95°C | 10 Minutes |
| Cycle (40 Cycles) | 95°C | 15 Seconds |
| | 60°C | 60 Seconds |

Using the CopyCaller software (manufactured by Applied Biosystems), the copy numbers of the target regions were evaluated using TaqMan Copy Number Assay probe and primer sets. Assuming that the copy numbers of the target regions in the reference DNA are 2, which has been adjusted by the TaqMan Copy Number Assay probe and primer (RNaseP, SEQ ID NO: 8), the relative copy numbers of the target regions in the test samples were also adjusted by the internal control (RNaseP, SEQ ID NO: 8) and calculated.

### [3] Identification of the nucleotide sequence of the PCR amplification product

In order to identify the nucleotide sequence of the PCR product amplified by quantitative PCR, the PCR product was cloned into the pGEM (R)-T Easy vector (manufactured by Promega K.K.) and a nucleotide sequence-detection sample was prepared using the BigDye Terminator Cycle Sequencing Kit (manufactured by Applied Biosystems). Then, the PCR product was sequenced by a DNA sequencer ABI PRISM(R) 3100 (manufactured by Applied Biosystems).

### 2-3 Results

It was revealed that there were seven chromosomal regions that enable the prediction of corpus uteri cancer susceptibility (7p22.3 [1,275,532 to 1,275,641] [SEQ ID NO: 1] within the UNCX gene, 9q22.32 [98,277,504 to 98,277,595] [SEQ ID NO: 2] within the PTCH1 gene, 14q22.1 [51,296,210 to 51,296,320] [SEQ ID NO: 3] within the NIN gene, 15q22.2 [59,732,253 to 59,732,342] [SEQ ID NO: 4] within the FAM81A gene, 15q26.1 [93,631,100 to 93,631,174] [SEQ ID NO: 5] within the RGMA gene, 19q12 [30,437,054 to 30,437,155] [SEQ ID NO: 6] within the C19orf2 gene, and 21q22.13 [38,122,020 to 38,122,129] [SEQ ID NO: 7] within the SIM2 gene). Table 2 shows the location information of those seven chromosomal regions based on NCBI; Feb. 2009 human reference sequence (GRCh37/hg19) (Table 2, see "chromosomal region"), nucleotide sequence information (Table 2, see "nucleotide sequence (5'-3')"), and primer set used for amplification (TaqMan Copy Number Assay probe and primer set) (Table 2, see "Copy number Assay ID", the website of Applied Biosystems [http://www5.appliedbiosystems.com/tools/cnv/]).

**[Table 2]**

| Copy number assay® ID | Nucleotide sequence (5'-3') | Chromosomal region (GRCh37/hg19) | Gene name |
|---|---|---|---|
| Hs03622829_ cn | | chr7: 1,275,532-1,275,641 | UNCX |
| Hs06834538_cn | | chr9: 98,277,504-98,277,595 | PTCH1 |
| Hs07054232_ cn | | chr14: 51,296,210 -51,296,320 | NIN |
| Hs07452491_cn | | chr15: 59,732,253 -59,732,342 | FAM81A |
| Hs05386131_ cn | | chr15: 93, 631, 100 -93,631,174 | RGMA |
| Hs07137112_cn | | chr19: 30,437,054 -30,437,155 | C19orf2 |
| Hs02291131_ cn | | chr21: 38, 122, 020 -38,122,129 | SIM2 |
| RNaseP (Internal control) | | chr14: 20,811,479 -20,811,565 | RNaseP |

[1] Figure 1 and Table 3 (Hs03622829_cn) : A DNA copy number of less than 0.5 in the chromosomal region (7p22.3 [1, 275, 532 to 1, 275, 641]), which is amplified by the primer and probe set (Hs03622829_cn), was observed in 12 out of 50 individuals (24.0%) in the group of healthy individuals, whereas that was observed in 41 out of 43 individuals (95.3%) in the group of corpus uteri cancer patients, revealing a significantly high frequency of DNA copy number of less than 0.5 (P < 0.0001, odds ratio of 64.9, Fisher's test) in the group of corpus uteri cancer patients.
[2] Figure 2 and Table 3 (Hs06834538_cn): A DNA copy number of less than 0.5 in the chromosomal region (9q22.32[98, 277, 504 to 98, 277, 595]), which is amplified by the primer and probe set (Hs06834S38_cn), was observed in 0 out of 50 individuals (0.0%) in the group of healthy individuals, whereas that was observed in 20 out of 43 individuals (46.5%) in the group of corpus uteri cancer patients, revealing a significantly high frequency of DNA copy number of less than 0.5 (P < 0.0001, odds ratio of 88.1, Fisher's test) in the group of corpus uteri cancer patients.
[3] Figure 3 and Table 3 (Hs07054232_cn) : A DNA copy number of less than 1.0 in the chromosomal region (14q22.1 [51, 296, 210 to 51, 296, 320]), which is amplified by the primer and probe set (Hs07054232_cn), was observed in 2 out of 50 individuals (4.0%) in the group of healthy individuals, whereas that was observed in 17 out of 43 individuals (39.5%) in the group of corpus uteri cancer patients, revealing a significantly high frequency of DNA copy number of less than 1.0 (P < 0.0001, odds ratio of 15.7, Fisher's test) in the group of corpus uteri cancer patients.
[4] Figure 4 and Table 3 (Hs07452491_cn): A DNA copy number of less than 1.5 in the chromosomal region (15q22.2 [59, 732, 253 to 59, 732, 342]), which is amplified by the primer and probe set (Hs07452491_cn), was observed in 8 out of 50 individuals (16.0%) in the group of healthy individuals, whereas that was observed in 37 out of 43 individuals (86.0%) in the group of corpus uteri cancer patients, revealing a significantly high frequency of DNA copy number of less than 1.5 (P < 0.0001, odds ratio of 32.4, Fisher's test) in the group of corpus uteri cancer patients.
[5] Figure 5 and Table 3 (Hs05386131_cn): A DNA copy number of less than 1.5 in the chromosomal region (15q26.1 [93, 631, 100 to 93, 631, 174]), which is amplified by the primer and probe set (Hs05386131_cn), was observed in 18 out of 50 individuals (36.0%) in the group of healthy individuals, whereas that was observed in 39 out of 43 individuals (90.7%) in the group of corpus uteri cancer patients, revealing a significantly high frequency of DNA copy number of less than 1.5 (P < 0.0001, odds ratio of 17.3, Fisher's test) in the group of corpus uteri cancer patients.
[6] Figure 6 and Table 3 (Hs07137112_cn) : A DNA copy number of less than 1.5 in the chromosomal region (19q12 [30, 437, 054 to 30, 437, 155]), which is amplified by the primer and probe set (Hs07137112_cn), was observed in 6 out of 50 individuals (12.0%) in the group of healthy individuals, whereas that was observed in 36 out of 43 individuals (83.8%) in the group of corpus uteri cancer patients, revealing a significantly high frequency of DNA copy number of less than 1.5 (P < 0.0001, odds ratio of 37.7, Fisher's test) in the group of corpus uteri cancer patients.
[7] Figure 7 and Table 3 (Hs02291131_cn): A DNA copy number of less than 1.5 in the chromosomal region (21q22.13 [38, 122, 020 to 38, 122, 129]), which is amplified by the primer and probe set (Hs02291131_cn), was observed in 7 out of 50 individuals (14.0%) in the group of healthy individuals, whereas that was observed in 35 out of 43 individuals (81.4%) in the group of corpus uteri cancer patients, revealing a significantly high frequency of DNA copy number of less than 1.5 (P < 0.0001, odds ratio of 26.9, Fisher's test) in the group of corpus uteri cancer patients.

From the analytical results of the quantitative PCR as described in [1] to [7] above, a corpus uteri cancer patient was successfully assessed as positive with significance in the group of corpus uteri cancer patients than in a group of healthy individuals by setting a cut-off value (a value set for assessing if an individual is a corpus uteri cancer patient or a healthy individual). The above results revealed that an individual having a DNA copy number polymorphism in the aforementioned seven chromosomal regions can be assessed as highly susceptible to corpus uteri cancer.

**[Table 3]**

| Copy number assay® ID | Cut-off value | Number of corpus uteri cancer patients (43 patients) | Non-cancer females (50 females) | Odds ratio | P Value |
|---|---|---|---|---|---|
| Hs03622829_cn | less than 0.5 | 41 (95.3%) | 12 (24.0%) | 64.9 | <0.0001 |
| Hs06834538_cn | less than 0.5 | 20 (46.5%) | 0 (0.0%) | 88.1 | <0.0001 |
| Hs07054232_cn | less than 1.0 | 17 (39.5%) | 2 (4.0%) | 15.7 | <0.0001 |
| Hs07452491_cn | less than 1.5 | 37 (86.0%) | 8 (16.0%) | 32.4 | <0.0001 |
| Hs05386131_cn | less than 1.5 | 39 (90.7%) | 18 (36.0%) | 17.3 | <0.0001 |
| Hs07137112_cn | less than 1.5 | 36 (83.8%) | 6 (12.0%) | 37.7 | <0.0001 |
| Hs02291131_cn | less than 1.5 | 35 (81.4%) | 7 (14.0%) | 26.9 | <0.0001 |

| | | | | | |
|---|---|---|---|---|---|
| The term "odds ratio" in the Table is an index indicating the relevance between the cause (DNA copy number polymorphism) and result (corpus uteri cancer), and the odds ratio is distributed from 0 to infinity. When the odds ratio is less than 1, an individual having the DNA copy number polymorphism can be assessed as less susceptible to corpus uteri cancer, whereas when the odds ratio is more than 1, an individual having the DNA copy number polymorphism can be assessed as highly susceptible to corpus uteri cancer. | | | | | |

Discriminant analysis was performed by selecting various combination patterns from the aforementioned seven chromosomal regions that enable the prediction of corpus uteri cancer susceptibility. A discriminant analysis was performed based on the results obtained in Figures 2, 3 and 6. In the analysis, a discriminant score Y can be obtained by using the linear discriminant formula (Y = [Hs06834S38_cn copy number] × (-4.7570) + [Hs07054232_cn copy number] × (3.1207) + [Hs07137112_cn copy number] × (-4.0499) + (7.4064) . When a discriminant score of 0 or more was determined as susceptible to corpus uteri cancer and a discriminant score of less than 0 was determined as not susceptible to corpus uteri cancer, among 43 true corpus uteri cancer patients, 39 individuals were assessed as "susceptible corpus uteri cancer" (a discriminant score of 0 or more) (sensitivity: 90.7%) by the above discriminant formula, while among 50 true healthy individuals, 46 individuals were assessed as "not having corpus uteri cancer" (a discriminant score of less than 0) (specificity: 92.0%). It is to be noted that the term "Hs06834538_cn copy number" refers to the number of copies of a chromosomal region amplified by the primer and probe set (Hs06834538_cn), that is, the number of copies of "9q22.32 (98, 277, 504 to 98, 277, 595)". Also, the terms "Hs07054232_cn copy number" refers to the number of copies of a chromosomal region amplified by the primer and probe set (Hs07054232_cn), that is, the number of copies of "14q22.1 (51, 296, 210 to 51, 296, 320)". Also, the terms "Hs07137112_cn copy number" refers to the number of copies of a chromosomal region amplified by the primer and probe set (Hs07137112_cn), that is, the number of copies of "19q12 (30, 437, 054 to 30, 437, 155)". From the above results, it was revealed that corpus uteri cancer was successfully identified with both sensitivity and specificity of over 90%, and also, both positive predictive value and negative predictive value of over 90% by performing discriminant analysis based on the results obtained by measuring DNA copy numbers in three chromosomal regions (9q22.32 [98,277,504 to 98,277,595], 14q22.1 [51,296,210 to 51,296,320], and 19q12 [30,437,054 to 30,437,155]). Furthermore, it was also revealed that the combination of the above three chromosomal regions was the combination of the smallest possible number of chromosomal regions that can identify corpus uteri cancer with both sensitivity and specificity of over 90% or both positive predictive value and negative predictive value of over 90%.

### Industrial Applicability

According to the present invention, the number of individuals who receive cancer screening such as a regular medical check-up, which is necessary for the prevention of corpus uteri cancer development and early detection of corpus uteri cancer, is expected to increase. Not only that, the number of individuals who receive diagnosis and treatment at an early stage of cancer is expected to increase, whereas mortality from corpus uteri cancer is expected to decrease.

## Claims

1. An assessment method of corpus uteri cancer susceptibility, comprising detecting each DNA copy number in a human chromosomal region consisting of a nucleotide sequence shown in any of SEQ ID NOs: 1 to 7 using a DNA extracted from blood of an individual subjected to assessment and a healthy individual being a control as a test sample, and comparing the copy numbers of both DNAs, wherein when the DNA copy number in the individual subjected to assessment is decreased compared to the DNA copy number in the healthy individual, it is assessed that the corpus uteri cancer susceptibility of the individual subjected to assessment is high.

2. The assessment method according to claim 1, comprising performing discriminant analysis by selecting two or more of DNA in a human chromosomal region consisting of a nucleotide sequence shown in any of SEQ ID NOs: 1 to 7 .

3. The assessment method according to claim 2, comprising selecting three DNAs consisting of nucleotide sequences shown respectively in SEQ ID NO: 2, SEQ ID NO: 3, and SEQ ID NO: 6.

4. The assessment method according to any one of claims 1 to 3, wherein the decrease in DNA copy number is detected by quantitative PCR.

## Patentansprüche

1. Verfahren zur Beurteilung der Anfälligkeit für ein Uteruskarzinom, welches die Erfassung der jeweiligen Anzahl von DNA-Kopien in einem menschlichen Chromosomenbereich bestehend aus einer Nukleotidsequenz, die in einer der SEQ ID Nummern 1 bis 7 unter Verwendung einer DNA gezeigt ist, die aus Blut einer der Beurteilung unterzogenen Person und einer gesunden Kontrollperson als eine Testprobe extrahiert worden ist, und das Vergleichen der Anzahl von Kopien beider DNA umfasst, wobei, wenn die Anzahl der DNA-Kopien in der der Beurteilung unterzogenen Person im Vergleich zu der Anzahl der DNA-Kopien in der gesunden Person verringert ist, die Anfälligkeit für ein Uteruskarzinom der der Beurteilung unterzogenen Person als hoch eingeschätzt wird.

2. Beurteilungsverfahren nach Anspruch 1, welches die Durchführung einer Diskriminanzanalyse umfasst, indem zwei oder mehr DNAs in einem menschlichen Chromosomenbereich bestehend aus einer Nukleotidsequenz ausgewählt werden, welche in einer der SEQ ID Nummern 1 bis 7 dargestellt ist.

3. Beurteilungsverfahren nach Anspruch 2, welches die Auswahl von drei DNAs umfasst, welche aus Nukleotidsequenzen bestehen, welche in der SEQ ID Nr. 2, SEQ ID Nr. 3 bzw. SEQ ID Nr. 6 dargestellt sind.

4. Beurteilungsverfahren nach einem der Ansprüche 1 bis 3, wobei die Verringerung in der Anzahl von DNA-Kopien durch quantitative PCR (Polymerase-Kettenreaktion) erfasst wird.

## Revendications

1. Procédé d'évaluation de la prédisposition au cancer du corps de l'utérus, comprenant les étapes consistant à détecter chaque nombre de copies d'ADN dans une région chromosomique humaine constituée d'une séquence de nucléotides représentée par l'une quelconque des séquences à numéro d'identification SEQ ID N° 1 à SEQ ID N° 7 en utilisant un ADN extrait du sang d'un individu soumis à l'évaluation et d'un individu bien portant servant de témoin en tant qu'échantillon d'essai, et à comparer les nombres de copies des deux ADN, où lorsque le nombre de copies d'ADN de l'individu soumis à l'évaluation est diminué par comparaison au nombre de copies d'ADN de l'individu bien portant, il est estimé que la prédisposition au cancer du corps de l'utérus de l'individu soumis à l'évaluation est forte.

2. Procédé d'évaluation selon la revendication 1, comprenant le fait de réaliser une analyse discriminante en sélectionnant deux ADN ou plus dans une région chromosomique humaine constituée d'une séquence de nucléotides représentée par l'une quelconque des séquences à numéro d'identification SEQ ID N° 1 à SEQ ID N° 7.

3. Procédé d'évaluation selon la revendication 2, comprenant le fait de sélectionner trois ADN constitués de séquences de nucléotides respectivement représentées par les séquences à numéro d'identification SEQ ID N° 2, SEQ ID N° 3, et SEQ ID N° 6.

4. Procédé d'évaluation selon l'une quelconque des revendications 1 à 3, dans lequel la diminution du nombre de copies d'ADN est détectée par PCR quantitative.


### SEQUENCE LISTING

<110> YAMAGUCHI UNIVERSITY
<120> Method for assessing endometrial cancer susceptibility
<130> FH24-011WO
<150> JP2012-094198
   <151> 2012-04-17
<160> 8
<170> PatentIn version 3.1
<210> 1
   <211> 110
   <212> DNA
   <213> Homo sapiens
<220>
   <223> Inventor: Yutaka, Suehiro; Kohsuke, Sasaki Inventor: Takae, Okada
<400> 1
   ggccggcgca caactcgcac ccgaccacgt gcagcggcga gcccatggac ccggaggaga     60
   tcgcgcgcaa ggagctggag aagatggaga agaagaagcg caagcacgag               110
<210> 2
   <211> 91
   <212> DNA
   <213> Homo sapiens
<400> 2
   gccacaatgg gtggctctaa atatttgata attggatcac tgaggtcttg aactaccaca     60
   atagtccttt ttcagcattc tgtgtgtgtg c                                   91
<210> 3
   <211> 111
   <212> DNA
   <213> Homo sapiens
<400> 3
   gccatacagc tcaactcctg aatttattac atcgaaacag attgcgtagt tgtcttttgt     60
   gactgacatg gtagctttgt aggatgagaa tcctgataaa gtccatcctg a             111
<210> 4
   <211> 90
   <212> DNA
   <213> Homo sapiens
<400> 4
   gctcttccgc ttctccttag tgctgcctcc aaaccaatgc accatcacca accaagtctc      60
   ctcacggaag gtagtgctat ctgcttgtgc                                       90
<210> 5
   <211> 74
   <212> DNA
   <213> Homo sapiens
<400> 5
   tccctggtac cgtcgatact cagagagctt ctgtagcctc cttcctggag gcgagggctg      60
   gttcccgcct ctcc                                                        74
<210> 6
   <211> 101
   <212> DNA
   <213> Homo sapiens
<400> 6
   ccacacattt tatggtatag gtcacagtct agacaggtta agcttccatt tttccaacaa      60
   atattttact gctgttagta aaaacagatt tagttaaaga c                         101
<210> 7
   <211> 110
   <212> DNA
   <213> Homo sapiens
<400> 7
   ccgtgattct tgaaaggtgt aggtttgatt actaggagat accaccgaca tttttcaata      60
   aagtactgca aaatgctttt gtgtctacct tgttattaac ttttggggct                110
<210> 8
   <211> 87
   <212> DNA
   <213> Homo sapiens
<400> 8
   ccctagtctc agaccttccc aagggacatg ggagtggagt gacagracgc actcagctcg      60
   tggccccact gatgagcttc cctccgc                                          87